# EUROPEAN PATENT APPLICATION

(11) **EP 2 110 372 A1**
(43) Date of publication of application: **21.10.2009**
(21) Application number: 08154656.6
(22) Date of filing: 16.04.2008
(51) Int. Cl.: C07D 207/38, C07C 59/125, C07C 69/67, C07C 235/06

(54) **Intermediates for the preparation of Pregabalin and process for their preparation**

(71) Applicant: Chemo Ibérica, S.A., 08028 Barcelona (ES)
(72) Inventor: Rasparini, Marcello, 27010, Cura Carpignano (PV) (IT); Tufaro, Roberto, 28060, Sozzago (Novara) (IT); Castaldi, Graziano, 28072, Briona (NO) (IT)
(74) Representative: Markvardsen, Peter

(57) **Abstract**

The present invention relates to intermediates useful for the preparation of Pregabalin and to a process for their preparation.

## Description

### Field of the invention

The present invention relates to intermediates useful for the preparation of Pregabalin and to a process for their preparation.

### Background of the invention

(±)-3-Aminomethyl-5-methyl-1-hexanoic acid (Pregabalin), which is also called β-isobutyl-γ-aminobutyric acid or isobutyl-GABA, is related to the endogenous inhibitory neurotransmitter γ-aminobutyric acid (GABA). GABA is one of the most widely distributed inhibitory neurotransmitters involved in the regulation of brain neuronal activity. The concentration of GABA is regulated by two pyridoxal 5'-phosphate dependent enzymes: L-glutamic acid decarboxylase (GAD), which catalyzes conversion of L-glutamic acid to GABA, and GABA aminotransferase, which degrades GABA to succinic semialdehyde. When the concentration of GABA diminishes below a threshold level in the brain, convulsions may result and, conversely, raising the GABA level appears to terminate seizures. The term "seizure" means excessive unsynchronized neuronal activity that disrupts normal brain function.

Pregabalin increases the concentration of GABA by activating GAD and is a potent anticonvulsant: it has the ability to suppress seizure while avoiding the undesirable side effect of ataxia. Pregabalin is useful as a therapeutic agent for the treatment of pain, epilepsy, convulsions, psychiatric disorders, attention deficit, hypersensitivity disorder, anxiety and mood disorders.

It has been discovered that the anticonvulsant effect of Pregabalin is dependent on its stereochemistry. The anticonvulsant effect of racemic Pregabalin is primarily attributable to the (S)-Pregabalin. (S)-Pregabalin shows better anticonvulsant activity, than the (R)-stereoisomer (see, for example, Yuen et al., Bioorganic & Medicinal Chemistry Letters, 1994, 4, 823).

(S)-Pregabalin is a compound of the following formula:

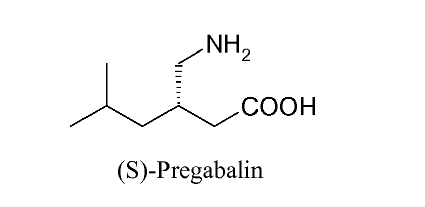

[(S)-(+)-3-aminomethyl-5-methyl-1-hexanoic acid], which is disclosed in EP 641 330 B1 and marketed under the trade name Lyrica®.

The commercial utility of Pregabalin requires an efficient, cost effective and safe method for preparing the S-enantiomer substantially free of the R-enantiomer in a large scale. Several methods have been used to prepare (S)-Pregabalin, *via* diastereomeric salt resolution or *via* an asymmetric synthesis.

The Patent EP 830 338 discloses a process for the resolution of racemic Pregabalin, which involves selective crystallization of a diastereoisomeric salt of racemic Pregabalin with the chiral resolving agent (S)-(+)-mandelic acid. The disclosed method requires the synthesis of 2-carboxyethyl-3-cyano-5-methylhexanoic acid ethyl ester, an intermediate of Pregabalin, by using a cyanide source (hydrogen cyanide, sodium cyanide, potassium cyanide, or magnesium cyanide).

The method described in the Patent Application EP 1 472 210, includes a process for producing substituted γ-amino acids, such as Pregabalin, where a substituted acrylic acid ester, (2E)-5-methylhex-2-ene carboxylic acid ethyl ester, is condensed with nitromethane to yield 5-methyl-3-nitromethylhexane carboxylic acid ethyl ester. Hydrogenation of 5-methyl-3-nitromethylhexane carboxylic acid ethyl ester to 4-isobutylpyrrolidin-2-one, with Nickel catalyst, and following hydrolysis, gave Pregabalin.

The method described in U.S. Patent 5,563,175 includes the use of the base *n*-butyllithium at low temperatures (< -35 °C) under carefully controlled conditions and the use of (4R,5S)-4-methyl-5-phenyl-2-oxazolidinone, as a chiral auxiliary, to introduce the desired stereochemical configuration in the final product.

(S)-Pregabalin may be prepared according to the process disclosed in EP 1 250 311, by an asymmetric hydrogenation of a cyano substituted olefin to produce a chiral cyano precursor of (S)-Pregabalin, which is further reduced to obtain (S)-Pregabalin. The application discloses the use of various C₂-symmetric bisphosphine ligands, including (R,R)-Me-DUPHOS, which result in substantial enrichment of (S)-Pregabalin over (R)-Pregabalin.

Thus, although these general strategies provide the target compound in high enantiomeric purity, they are not practical for large-scale synthesis, because they employ reagents which are either expensive or difficult to handle or both, as well as specialized equipment to reach the required operating conditions. These drawbacks make them ill-suited to be employed on common multipurpose industrial plants.

Because (S)-Pregabalin is being developed as a commercial pharmaceutical product, the need exists for an efficient, cost effective, and safe method for its large scale synthesis, that overcomes the limitations of the above procedures.

An object of the present invention is to provide intermediates for the preparation of Pregabalin and an efficient, economical and commercially useful process for preparing these intermediates that avoids the above identified problems.

### Summary of the invention

An intermediate which is useful for preparing Pregabalin is a compound of formula (I): 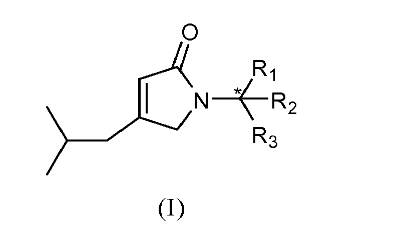 wherein R₁, R₂ and R₃ are each independently hydrogen, C₁-C₆ alkyl, C₃-C₆ cycloalkyl, aryl, heteroaryl, optionally substituted. This intermediate can simply be converted into the known drug substance, Pregabalin, by a method described in our EP pending patent application 07119355.

The present invention relates to a process and intermediates for preparing a compound of formula (I), both as a mixture of (*R*,*S*) enantiomers, or as single (*R*) or (*S*) enantiomer.

The invention provides a process for preparing a compound of formula (I), 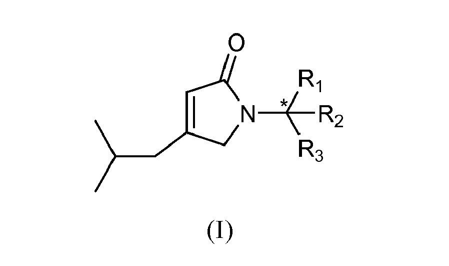 wherein R₁, R₂ and R₃ are as defined above;
which comprises the following steps:
a) transforming a compound of formula (VII) 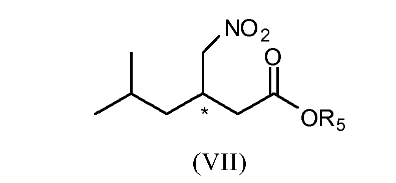 wherein R₅ is a C₁-C₄ alkyl group, or aryl,
   into an ester of formula (VI) 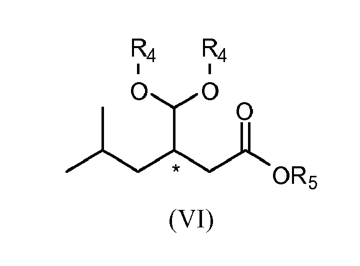 wherein R₄ is a C₁-C₆ alkyl group and R₅ is as defined above;
b) hydrolysing the ester of formula (VI) into a carboxylic acid of formula (III) 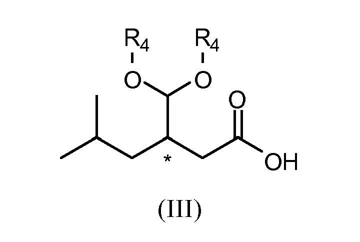 wherein R₄ is as defined above;
c) transforming the carboxylic acid of formula (III) into an amide of formula (II) 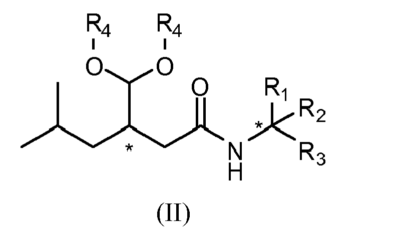 wherein R₁, R₂, R₃ and R₄ are as defined above;
d) cyclizing the amide of formula (II), into a compound of formula (I).

In another embodiment, the invention provides a compound of formula (II), as defined above, or a hydrate, a solvate thereof.

In another embodiment, the invention provides a compound of formula (III), as defined above, or a salt, a hydrate, a solvate thereof.

In another embodiment, the invention provides a compound of formula (VI), as defined above, or a hydrate, a solvate thereof.

In another embodiment, the invention provides the use of compounds of formula (I), (II), (III) or (VI) as intermediates in the preparation of Pregabalin.

The present method is conducted under mild conditions and it makes use of generally inexpensive, readily available and safe reagents. The present invention offers several advantages over previous methods of making intermediates of Pregabalin. For example, the present process does not require the use of hazardous cyano compounds, the carcinogenic acrylonitrile, or low temperatures, as required in previous methods.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "C₁-C₄ alkyl" refers to a straight or branched, saturated or unsaturated alkyl chain, having from 1 to 4 carbon atoms. Examples of alkyl groups include, without limitation, methyl, ethyl, *n*-propyl, isopropyl, *n*-butyl, *sec*-butyl, isobutyl, *tert*-butyl, vinyl, allyl, and the like. A preferred C₁-C₄ alkyl group of the present invention is methyl or ethyl, more preferably ethyl.

The term "C₁-C₆ alkyl" refers to a straight or branched hydrocarbon having from 1 to 6 carbon atoms, preferably 1 to 4 carbon atoms, more preferably up to 2 carbon atoms. Examples of alkyl groups include, without limitation, methyl, ethyl, n-propyl, isopropyl, *n-*butyl, *sec*-butyl, isobutyl, *tert*-butyl, *n*-pentyl, *n*-hexyl groups and the like. The C₁-C₆ alkyl group may be optionally substituted. A preferred C₁-C₆ alkyl group of the present invention is methyl, ethyl, isopropyl.

The term "C₃-C₆ cycloalkyl" refers to a saturated or unsaturated carbocyclic group of from 3 to 6 carbon atoms having a single ring (*e*.*g*., cyclopropyl, cyclobutyl, cyclopentyl, cyclohexyl or cyclohexenyl). The C₃-C₆ cycloalkyl group may be optionally substituted. Examples of C₃-C₆ cycloalkyl include, without limitation, cyclopentyl, cyclohexyl, and the like.

The term "aryl" refers to an aromatic carbocyclic group having a single ring (e.g., phenyl), multiple rings (e. g., biphenyl), or multiple condensed rings in which at least one is aromatic (e.g., 1,2,3,4-tetrahydronaphthyl, naphthalen-1-yl, 2-naphthyl, anthryl, or phenanthryl). The aryl group may be optionally substituted. A preferable aryl group of the present invention is phenyl, *p*-nitrophenyl, naphthalen-1-yl or 2-naphtyl, more preferably phenyl or naphthalen-1-yl.

The term "heteroaryl" refers to a monocyclic heteroaromatic, or a bicyclic or a tricyclic fused-ring heteroaromatic group. The heteroaryl group may be optionally substituted. Examples of heteroaromatic groups include, without limitation, pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "substituted" refers to groups in which one or more hydrogen atoms have been replaced with one or more substituents selected from the group consisting of C₁-C₆ alkyl, C₃-C₆ cycloalkyl, aryl, heteroaryl, halogen, amino, aminosulfonyl, ammonium, aminocarbonyl, sulfinyl, sulfanyl, sulfonyl, hydroxy, alkoxy, carboxy, alkoxycarbonyl, carbamate, trihalomethyl, cyano, mercapto, nitro, and the like.

The compounds prepared by the process of the present invention may have one or more stereogenic centers and may exist and may be used or isolated in optically active (enantiomeric or diastereomeric) and racemic forms. It is to be understood that the processes of the present invention can give rise to any racemic or optically-active forms, or mixtures thereof. It is to be further understood the products of the invention process can be isolated as racemic, or optically active forms, or mixtures thereof. Purification and characterization procedures for such products are known to those of ordinary skill in the art, and include recrystallization techniques, as well as chiral chromatographic separation procedures as well as other methods.

The formulae include one or more "*" (asterisk) to indicate stereogenic (chiral) center, although the absence of asterisks does not indicate that the compound lacks one stereocenter. Such formulae may refer to the racemate or to individual enantiomers or diastereomers, which may or may not be substantially pure.

The term "racemic" refers to a sample of a chiral compound which contains both the (+) and (-) isomers in equal amounts.

A mixture of (*R*,*S*) enantiomers can contain the two single enantiomers in any ratio to each other. The enantiomeric purity is generally expressed as "enantiomeric excess" and defined, for example, for the (*S*) enantiomer as (*S*-*R*)/(*R*+*S*)x100, wherein *S* and *R* are respectively the amounts of the (*S*) and (*R*) enantiomers.

The term "single (*S*) or (*R*) enantiomer" means that the enantiomeric purity is usually at least about 96%, preferably at least about 99%.

The term "Pregabalin" refers to racemic-Pregabalin, or (S)-Pregabalin.

The term "(S)-Pregabalin" refers to enantiomerically pure (S)-Pregabalin, or enantiomerically enriched (S)-Pregabalin.

The term "about" encompasses the range of experimental error that may typically occur in a measurement.

Those compounds prepared according to the present invention that are acidic in nature are capable of forming base salts with various pharmacologically acceptable cations.

Examples of such salts include the alkali metal or alkaline earth metal salts and, particularly, the calcium, magnesium, sodium and potassium salts or ammonium, for example *tert*-butylammonium and substituted ammonium or amino acids salts, for example lysine, of the compounds of the present invention.

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (*e*.*g*., ethanol).

Generally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible and inert solvents, which may influence the reaction rate and yield. Depending on the nature of the reactants, the one or more solvents may be polar protic solvents, polar aprotic solvents, non-polar solvents, or some combination. Suitable solvents are water; an alcohol, such as methanol, ethanol, *i*-propanol, 1- or 2- or *tert*-butanol; an ester, such as ethyl acetate or isopropyl acetate; an ether, such as tetrahydrofuran, 2-methyltetrahydrofuran or 1,4-dioxane; an aliphatic or aromatic hydrocarbon, such as hexane or heptane, toluene or xylenes; dimethylformamide, dimethylacetamide or N-methylpyrrolidone; or mixtures thereof.

The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

The process of the invention for making compounds of formula (I) is illustrated in the following reactions Scheme 1. 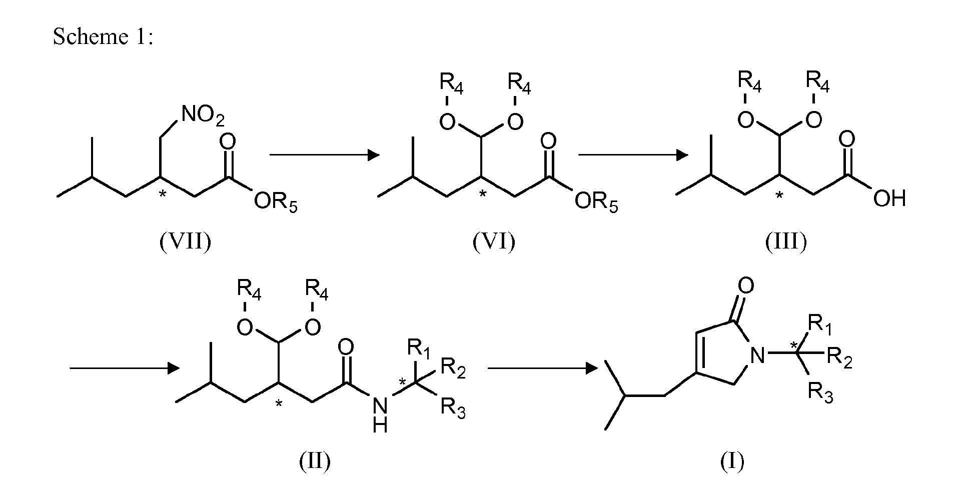 wherein:
R₁, R₂ and R₃ are each independently hydrogen, C₁-C₆ alkyl, C₃-C₆ cycloalkyl, aryl, heteroaryl, optionally substituted;
R₄ is a C₁-C₆ alkyl group;
R₅ is a C₁-C₄ alkyl group, or aryl.

According to a preferred embodiment, a compound of formula (VII) is transformed with a Nef reaction into an ester of formula (VI); that is hydrolysed into a carboxylic acid of formula (III). Transforming the carboxylic acid of formula (III) into an amide of formula (II) and cyclizing the amide of formula (II), a compound of formula (I) is obtained.

In one step of the present method for making a compound of formula (I), the nitro compound (VII) is transformed into an ester of formula (VI), by addition of a suitable base in a suitable alcoholic solvent to form a nitronate salt. The nitronate salt is formed at a temperature comprised from about 0 °C to 25 °C, preferably from about 0 °C to 15 °C. A suitable base is for example an alcoholate salt, such as sodium or potassium methoxide, sodium or potassium ethoxide, sodium or potassium t-butoxide. The alcoholate is added in a ratio to the nitro compound (VII) comprised from about 1.0 to 1.5 molar equivalents, preferably from about 1.1 to 1.2 molar equivalents. A suitable alcoholic solvent is methanol, ethanol, 1-propanol, 1-butanol, ethylene glycol, propylene glycol or 2,2-dimethylpropane-1,3-diol or mixtures thereof, preferably methanol or ethanol.

The said nitronate salt is subsequently added to a solution of a mineral acid, such as hydrochloric acid or sulfuric acid, and dissolved in the same alcoholic solvent used for the nitronate salt formation. A preferred mineral acid is concentrated sulfuric acid, at a concentration ranging from about 90 to 100%, preferably from about 95% to 98%. The preferred mineral acid is employed in a molar ratio to compound (VII) comprised from about 1.0 to 5.0 molar equivalents, preferably from about 1.0 to 2.0 molar equivalents. The addition of the nitronate salt solution to the acidic solution is carried out at a temperature comprised from about -25 °C to 25 °C, preferably from about -20 °C to 0 °C. After complete addition, the mixture is neutralized by addition of the same base used to form the nitronate salt, followed by removal of the inorganic salts and optional concentration of the solution of the ester of formula (VI).

The compound of formula (VII), wherein R₅ is ethyl, is a known compound described in US 7141695. The nitro compound of formula (VII) may be prepared by reacting malonic acid mono ester with isovaleraldehyde, in the presence of a base, such as 1,4-dimethylaminopyridine, in a polar aprotic solvent, such as dimethylformamide, dimethylacetamide. The mixture is stirred at room temperature for 24 hours, then diluted with aqueous bicarbonate and an aromatic hydrocarbon, such as toluene or xylenes, to isolate an intermediate that was successively reacted with nitromethane and an organic base, such as 1,8-diazabicyclo[5.4.0]undec-7-ene (DBU), 1,4-diazabicyclo[2.2.2]octane (DABCO), N,N,N',N'-tetramethylguanidine, to give a compound of formula (VII).

In another step of the present method, the ester of formula (VI) is hydrolysed to a carboxylic acid of formula (III) by an alkaline hydrolysis reaction, followed by acidification and extraction in a suitable inert solvent. The alkaline hydrolysis reaction is carried out by any method known in the art. The acidification step is carried out by addition of a diluted mineral or organic acid, such as hydrochloric acid, sulfuric acid or acetic acid. A suitable inert solvent is for example an ether, such as diethyl ether, t-butyl methyl ether; an aromatic hydrocarbon, such as toluene or xylenes.

The compounds of the general formula (VI) and (III) thus obtained are novel.

In another embodiment, the present invention provides a compound of the general formula (VI), as defined above, as well as its hydrates, solvates, racemic forms, enantiomers, diastereomers, and polymorphs, and mixtures thereof.

Preferred examples of the compounds of formula (VI) are compounds wherein R₄ is methyl, ethyl or isopropyl and R₅ is methyl or ethyl.

In another embodiment, the present invention provides a compound of the general formula (III), as defined above, as well as its hydrates, solvates, racemic forms, enantiomers, diastereomers, and polymorphs, and mixtures thereof, and the pharmaceutically acceptable salts thereof.

Preferred examples of the compounds of formula (III) are compounds wherein R₄ is methyl, ethyl or isopropyl.

Preferred examples of the compounds of formula (III) are selected from:
3-(dimethoxymethyl)-5-methylhexanoic acid;
3-(diethoxymethyl)-5-methylhexanoic acid;
3-(diisopropoxymethyl)-5-methylhexanoic acid.

In another step of the present method, the carboxylic acid of formula (III) is transformed into an amide of formula (II), with any coupling agent, such as carbonyldiimidazole (CDI), dicyclohexyl carbodiimide (DCC), N-(3-dimethylaminopropyl)-N'-ethylcarbodiimide (EDC); or a carboxy-activating reagent employed in the formation of amide or peptide bonds well known to those skilled in the art.

A particularly suitable and industrially applicable method for activating a carboxylic acid to form an amide is first to transform the carboxylic acid of formula (III) into a mixed anhydride by treatment with an acyl halide of formula (IIIa) 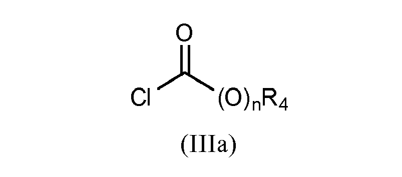 wherein R₄ is as defined above and n is 0 or 1.

A preferred acyl halide of formula (IIIa) is an acid chloride, for example acetyl chloride, propionyl chloride or pivaloyl chloride; or a chloroformate, such as ethyl chloroformate or isobutyl chloroformate. More preferably, the acyl halide is pivaloyl chloride or ethyl chloroformate. The reaction for activating a carboxylic acid of formula (III) is carried out in the presence of an organic base, in an inert solvent, to obtain an activated derivative of formula (IV) 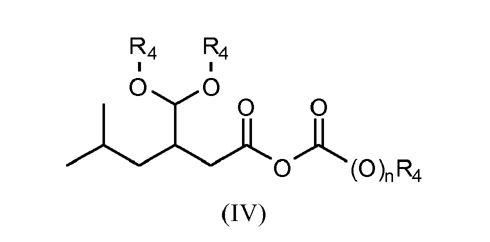 wherein R₄ and n are as defined above, which typically is not isolated from the reaction mixture.

Any suitable organic base known to those skilled in the art can be used. A suitable organic base is, for example, a tertiary amine, such as triethylamine, tributylamine, *N*,*N-*diisopropylethylamine, *N*-methylmorpholine, preferably triethylamine.

Any suitable solvent known to those skilled in the art can be used for activating the carboxylic acid of formula (III). Suitable solvents are a polar aprotic solvent, such as an ether, for example tetrahydrofuran, 2-methyltetrahydrofuran or 1,4-dioxane; or a polar protic solvent, such as dimethylformamide, dimethylacetamide; or mixtures thereof. Preferably the solvent is tetrahydrofuran.

The carboxy-activating regent is employed in 1:1 molar ratio to the carboxylic acid of formula (III), and the organic base is employed in 1.0 to 2.0 molar equivalents relative to the carboxylic acid of formula (III), preferably from 1.2 to 1.5 molar equivalents.

Optionally, the reaction is carried out in the presence of an acylation catalyst, such as 4-(dimethylamino)pyridine. The acylation catalyst is added in 0.01 to 0.1 molar equivalents relative to the carboxylic acid of formula (III). The activation reaction is carried out at a temperature ranging from about -10 °C to 25 °C, preferably from about 0 °C to 20 °C.

A compound of formula (IV) is further straight transformed, without isolation, into an amide of formula (II), 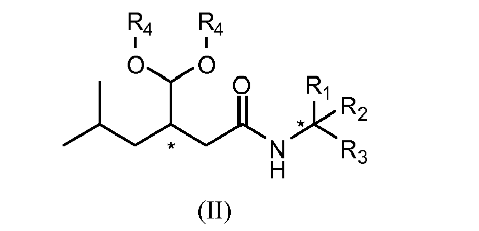 wherein R₁, R₂, R₃ and R₄ are as defined above, either as a mixture of (*R,R*) and (*R*,*S*) diastereoisomers, or (S,R) and (*S*,*S*) diastereoisomers or single (*R,R*) or (*R*,*S*) or (*S,R*) or (*S*,*S*) diastereoisomers; by addition of a primary amine of formula (V) 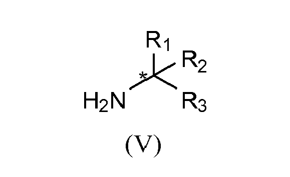 wherein R₁, R₂ and R₃ are as defined above.

The primary amine of formula (V) used in the process of the present invention may have one or more stereogenic centers and may exist and may be used in optically active or racemic forms. It is to be understood that the process of the present invention can employ any racemic, optically active, or stereoisomeric form, or mixtures thereof, of a primary amine of formula (V).

Examples of a primary amine of formula (V), include, without limitation, methylamine, ethylamine, n-propylamine, isopropylamine, *n*-butylamine, isobutylamine, *tert*-butylamine, triphenylmethylamine, 1-methyl-1-phenylethylamine, benzylamine, (R)-, (S)- or (*rac*)-1-phenylethylamine, (R)-, (S)- or (*rac*)-1-(naphthalen-1-yl)ethylamine, (R)- or (S)- or (*rac*)-2-napht-1-ylethylamine, (R)-, (S)-or (*rac*)-2-phenylglycinol or any isomer of 1-amino-2-indanol. Preferably the primary amine of formula (V) is methylamine, ethylamine, *n*-propylamine, *tert*-butylamine, benzylamine, (R)- or (S)-1-phenylethylamine or (R)- or (S)-1-(naphthalen-1-yl)ethylamine; more preferably benzylamine, (R)- or (S)-1-phenylethylamine or (R)-or (S)-1-(naphthalen-1-yl)ethylamine.

The primary amines described above are commercially available compounds.

The reaction of the primary amine of formula (V) and a compound of formula (IV) is carried out in the same solvent used in the activation step, at a temperature range from about 0 °C to the reflux temperature of the reaction mixture, preferably between about 0 °C and 25 °C, more preferably at about 25 °C. The molar ratio primary amine (V)/carboxylic acid (III) is comprised from 1.0 to 2.0 molar equivalents, preferably from 1.0 to 1.5 molar equivalents, more preferably from 1.0 to 1.2.

The amide-forming reaction is optionally carried out by addition of an organic base, such as a tertiary amine, for example triethylamine, tributylamine, *N*,*N-*diisopropylethylamine, *N*-methylmorpholine, preferably triehylamine.

The compounds of formula (II) are novel.

In another embodiment, the present invention provides a compound of formula (II), as defined above, as well as its hydrates, solvates, racemic forms, diastereomers, as a mixture of (*R,R*) and (*R*,*S*) diastereoisomers, or (*S*,*R*) and (*S*,*S*) diastereoisomers or single (*R,R*) or (*R*,*S*) or (*S,R*) or (*S*,*S*) diastereoisomers, and polymorphs. Preferably a compound of formula (II), is in the form of a mixture of (*R,R*) and (*R*,*S*) or (*S*,*R*) and (*S*,*S*) diastereoisomers, typically with at least about 96%, more preferably at least about 99% enantiomeric purity referred to the stereocenter directly attached to the nitrogen atom.

Preferred examples of compounds of formula (II) are compounds wherein:
R₁, R₂ and R₃ are each independently hydrogen, C₁-C₆ alkyl or aryl, optionally substituted; more preferably R₁, R₂ and R₃ are each independently hydrogen, methyl, phenyl or 1-(naphthalen-1-yl);
R₄ is methyl, ethyl or isopropyl.

Preferred examples of the compounds of formula (II) are selected from:
3-(dimethoxymethyl)-5-methyl-N-((*R*)-1-phenylethyl)hexanamide;
3-(diethoxymethyl)-5-methyl-N-((*R*)-1-phenylethyl)hexanamide;
3-(diisopropoxymethyl)-5-methyl-N-((*R*)-1-phenylethyl)hexanamide;
3-(dimethoxymethyl)-5-methyl-N-((*R*)-1-(naphthalen-1-yl)ethyl)hexanamide;
3-(diethoxymethyl)-5-methyl-N-((*R*)-1-(naphthalen-1-yl)ethyl)hexanamide;
3-(diisopropoxymethyl)-5-methyl-N-((*R*)-1-(naphthalen-1-yl)ethyl)hexanamide;
3-(dimethoxymethyl)-5-methyl-N-((*S*)-1-phenylethyl)hexanamide;
3-(diethoxymethyl)-5-methyl-N-((*S*)-1-phenylethyl)hexanamide;
3-(diisopropoxymethyl)-5-methyl-N-((*S*)-1-phenylethyl)hexanamide;
3-(dimethoxymethyl)-5-methyl-N-((*S*)-1-(naphthalen-1-yl)ethyl)hexanamide;
3-(diethoxymethyl)-5-methyl-N-((*S*)-1-(naphthalen-1-yl)ethyl)hexanamide;
3-(diisopropoxymethyl)-5-methyl-N-((*S*)-1-(naphthalen-1-yl)ethyl)hexanamide.

In another step of the present method, the compound of formula (II) is cyclized to obtain a compound of formula (I), as defined above, both as a mixture of (*R*,*S*) enantiomers, or as single (*R*) or (*S*) enantiomer. Preferably a compound of formula (I) is in the form of the single (*R*) or (*S*) enantiomer, in particular as single (*R*) enantiomer, typically with at least about 96%, more preferably at least about 99% enantiomeric purity.

Preferred examples of compounds of formula (I) are compounds wherein R₁, R₂ and R₃ are each independently hydrogen, C₁-C₆ alkyl or aryl, optionally substituted; more preferably R₁, R₂ and R₃ are each independently hydrogen, methyl, phenyl or 1-(naphthalen-1-yl).

Preferred examples of the compounds of formula (I) are selected from:
(*R*)-4-isobutyl-1-(1-phenylethyl)-1H-pyrrol-2(5H)-one;
(*S*)-4-isobutyl-1-(1-phenylethyl)-1H-pyrrol-2(5H)-one;
(*R*)-4-isobutyl-1-(1-(naphthalen-1-yl)ethyl)-1H-pyrrol-2(5H)-one;
(*S*)-4-isobutyl-1-(1-(naphthalen-l-yl)ethyl)-1H-pyrrol-2(5H)-one.

The cyclization of a compound of formula (II) can be carried out by reaction with a suitable acid in a solvent. A suitable acid is a mineral acid, such as hydrochloric or sulfuric acid; or an organic acid, such as carboxylic acid, for instance acetic acid, trichloroacetic acid or trifluoroacetic acid; or a sulfonic acid, for instance *p*-toluenesulfonic acid or camphorsulfonic acid. Preferably the acid is a carboxylic acid or a sulfonic acid; more preferably a sulfonic acid, more and more preferably a camphorsulfonic acid.

The amounts of acid that can be used is from about 0.01 to 0.1 equivalents to compound of formula (II), preferably from 0.05 to 0.1 equivalents, in an aprotic solvent, such as C₆-C₈ alkanes, preferably hexane or heptane, or mixtures thereof; or in an aromatic solvent, such as benzene, toluene or xylenes, preferably toluene; at a temperature ranging from about 50 °C to the reflux temperature of the reaction mixture, preferably at reflux temperature.

Compounds of formula (I) are intermediates for the preparation of pharmaceutically effective Pregabalin.

In another embodiment, the invention provides use of compounds of formula (I), (II), (III) or (VI), as defined above, as intermediates in the preparation of Pregabalin.

Many of the compounds described in this disclosure, are capable of forming pharmaceutically acceptable salts. These salts include, without limitation, acid addition salts (including diacids) and base salts.

It is contemplated that the compounds of the present method can be found or isolated in the form of hydrates or solvates, in different polymorphic forms, i.e., different crystalline forms, which are considered to fall within the scope of the present invention.

The process of the invention for the preparation of the above described pregabalin intermediates is particularly advantageous for the production on an industrial scale. In fact, all the starting materials are cheaply available in bulk and do not pose particular safety or toxicity concerns. All the steps proceed with >80% yield, thus attaining a reduction in the by-products and waste streams and providing the desired intermediates in high yields and satisfactory purity.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
THF (tetrahydrofuran), MTBE (tert-butyl methylether), DMAP (4-dimethylaminopyridine), m.p. (melting point).

### Example 1

### Ethyl 5-methyl-3-(nitromethyl)hexanoate (VIIa)

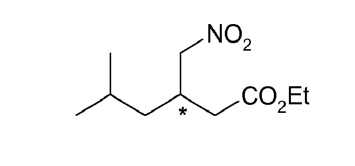

A 1000 mL three-necked round bottomed flash fitted with nitrogen inlet, reflux condenser and thermometer, was charged with malonic acid monoethyl ester (52.5 g, 0.397 mol), dimethylformamide (500 mL), isovaleraldehyde (32.8 mL, 0.305 mol) and 1,4-dimethylaminopyridine (3.73 g, 0.031 mol). The mixture is magnetically stirred at 15-20 °C for 24 hours, then diluted with aqueous bicarbonate (500 mL) and toluene (350 mL). After stirring and phase separation, the organic layer was washed twice with bicarbonate solution (100 mL), then with 1 N HCl in water (50 mL). The organic layer was dried over sodium sulfate, filtered and concentrated to a residue (41.5 g), as a colourless liquid, containing 8% w/w of toluene, as detected by ¹H NMR.
Yield: 80%.
To the residue was added nitromethane (44.7 g, 0.732 mol) and N,N,N',N'-tetramethylguanidine (30.9 g, 0.268 mol) and the mixture was stirred at 25 °C for 18 hours, then diluted with toluene (250 mL) and washed twice with water (100 mL) and twice with 1 N HCl in water (150 mL). Toluene was removed under reduced pressure to obtain the title product, as a light yellow oil (47.7 g).
Yield: 90%.
¹H NMR (300 MHz, CDCl) 0.91 (t, J=5.8 Hz, 2 x 3H, (CH₃)₂CH)), 1.26 (t, J=7.3 Hz, 3H, CH₃CH₂O), 1.65 (septet, J=6.7 Hz, 1H, CHMe₂), 2.41 (d, J=6.1 Hz, 2H, CH₂CO₂Et), 2.66 (septet, J=6.7, 1H, CHCH₂NO₂), 4.14 (q, J=7.3, 2H, CH₃CH₂O), 4.39-4-52 (m, 2H, CH₂NO₂)

### Example 2

### Methyl 3-(dimethoxymethyl)-5-methylhexanoate and ethyl 3-(dimethoxymethyl)-5-methylhexanoate mixture (VIa)

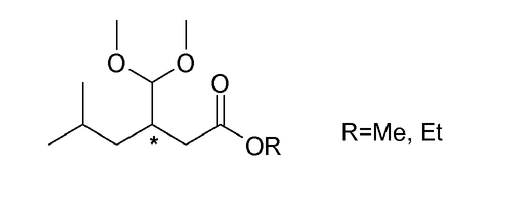

In a two necked 500 mL round bottomed flask (FLASK "A"), fitted with nitrogen inlet and thermometer, methanol (200 mL) and ethyl 5-methyl-3-(nitromethyl)hexanoate (VIIa) (40.0 g, 184 mmol), as obtained in Example 1, are charged. The solution is cooled in a ice-water bath and sodium methoxide 30% w/w in methanol (39.8 g, 221 mmol) is added, keeping the temperature below 25 °C.

In a three necked 500 mL round bottomed flask (FLASK "B"), fitted with nitrogen inlet, thermometer and a glass stopper, methanol (200 mL) is charged. The solution is cooled to -15 °C in a acetone-ice bath and sulfuric acid 98% (43.3 g, 432 mmol) is added, keeping the temperature below -10 °C. After complete addition, the mixture is cooled to -20 °C. The glass stopper is replaced by an addition funnel charged with the solution of FLASK A and the nitronate solution is added to sulfuric acid-methanol, keeping the temperature below -10 °C. After complete addition the ice bath is removed and the temperature allowed to reach 20 °C in about 1 hour. The resultant pale yellow slurry is cooled again to -10 °C and added with sodium methoxide 30% w/w in methanol (113 g, 628 mmol). The temperature is kept below 10 °C and the pH is checked periodically during the addition. The final pH value should be around 7. If the pH is higher than 7 it can be corrected by addition of acetic acid. To the resulting yellow slurry, toluene is added (200 mL) and the mixture toluene-methanol is evaporated under reduced pressure to eliminate most of methanol. The slurry is diluted again with toluene (200mL) and filtered on a thin Celite pad. The filtrate is concentrated to dryness, the residue is distilled under vacuum (2-3 mmHg) with a short path distillation apparatus, collecting a single fraction with a boiling range of 85-90 °C, obtaining the title product (27.4 g), as about 1:1 mixture of ethyl and methyl esters.
Yield: 66%.

The ethyl ester can be transesterified by treating the esters mixture in methanol in the presence of catalytic sulfuric acid.
¹H NMR (methyl ester) (300 MHz, CDCl₃) 0.89 (t, J=6.4 Hz, 2 x 3H, (CH₃)₂CH), 1.07-1.17 & 1.27-1.36 (2 x m, 2 x 1H, (CH₃)₂CHCH₂), 1.55-1.65 (m, 1H, (CH₃)₂CH), 2.19-2.44 (m, 2H, CH₂CO₂Me), 3.34 & 3.36 (2 x s, 2 x 3H, CH₃O), 3.65 (s, 3H, CO₂CH₃), 4.16 (d, J=4.9 Hz, CH(OMe)₂)

### Example 3

### 3-(Dimethoxymethyl)-5-methylhexanoic acid (IIIb)

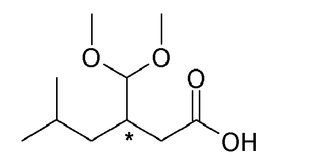

The esters mixture (VIa) (10.0 g, about 44 mmol), as obtained in Example 2, was dissolved in methanol (50 mL) and water (20 mL) and treated with 50% sodium hydroxide (5.0 g, 62.5 mmol). After 6 hours stirring at 35 °C, methanol was removed under reduced pressure, MTBE (100 mL) was added and the pH was adjusted to 4 by addition of IN HCl, while vigorously stirring. After phase separation and drying over sodium sulphate, the solvent was removed under reduced pressure to obtain the title acid (8.2 g) as a almost colourless oil.
Yield: 90%.
¹H NMR (300 MHz, CDCl₃) 0.88 (t, J=6.4 Hz, 2 x 3H, (CH₃)₂CH), 1.14-1-31 (m, 2 1H, (CH₃)₂CHCH₂), 1.58-1.61 (m, 1H, (CH₃)₂CH), 2.24-2.30 & 2.39-2.45 (m, 2H, CH₂CO₂Me), 3.34 & 3.36 (2 x d, J=3.7 Hz, 2 x 3H, CH₃O), 4.18 (d, J=4.9 Hz, CH(OMe)₂), 10.4 (bs, 1H, CO₂H)

### Example 4

### 3-(Dimethoxymethyl)-5-methyl-N-((R)-1-phenylethyl)hexanamide (IIa)

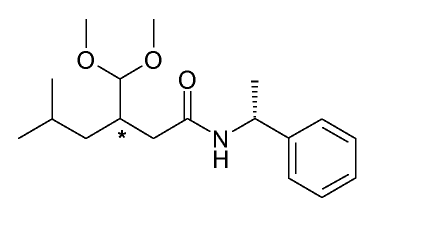

The acid (IIIb) (2.0 g, 9.8 mmol), as obtained in Example 3, was dissolved in THF (20 mL), in a 100 mL three-necked flask fitted with thermometer, nitrogen inlet and a rubber septum. The mixture was cooled to 0 °C and DMAP (120 mg, 0.98 mmol) and triethylamine (1.6 mL, 11.8 mmol) were added, followed by pivaloyl chloride (1.2 mL, 9.8 mmol). The mixture was warmed to 20 °C over 2 hours, then triethylamine (1.4 mL, 9.8 mmol) was added, followed by (*R*)-1-phenylethylamine (Va) (1.5 mL, 11.8 mmol). The reaction mixture was stirred at room temperature overnight, then THF was removed under reduced pressure, and the remaining aqueous phase was extracted with toluene. The organic layer was washed with water, brine and dried over sodium sulfate. Toluene was removed under reduced pressure to afford the title product (2.5 g) as a yellow viscous oil.
Yield: 83%.
¹H NMR (300 MHz, CDCl₃) 0.86 (t, J=6.4 Hz, 2 x 3H, (CH₃)₂CH), 1.21-1.26 (m, 2H, (CH₃)₂CHCH₂), 1.45 (d, J= 7.0 Hz, CH₃CHN), 1.52-1.62 (m, 1H, (CH₃)₂CH), 2.08-2-28 (m, 2H, CH₂CON), 3.33 & 3.34 (2 x s, 2 x 3H, CH₃O), 4.11-4.14 (m, 1H, CH(OMe)₂), 5.10 (quin, J=7.0 Hz, NCH), 7.29-7.31 (m, 5H, ArH)

### Example 5

### (S)-4-Isobutyl-1-(1-phenylethyl)-1H-pyrrol-2(5H)-one (Ia)

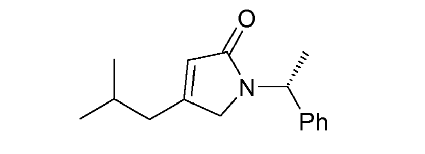

The amide (IIa) (2.0 g, 6.5 mmol), as obtained in Example 4, was dissolved in toluene (20 mL), and camphorsulfonic acid (150 mg, 0.65 mmol) was added. The solution was heated at reflux under nitrogen atmosphere for 1 hour, then cooled to room temperature. The reaction mixture was diluted with toluene, washed with aqueous sodium bicarbonate, brine and then dried over sodium sulfate. Toluene was removed under reduced pressure and the residue taken up in hexane and crystallized with cooling in a ice bath, obtaining the title compound (1.2 g) as colourless crystals, m.p. 61-62 °C.

Yield: 76%.
¹H NMR (300 MHz, CDCl₃): 0.86-0.89 (m, 6H), 1.58 (d, J=7.0, 3H), 1.78 (nonuplet, J=6.7, 1H), 2.17 (dd, J=6.7, 1.2, 2H), 3.45 (dd, J=19.0, 1.2, 1H), 3.76 (dd, J=19.0, 1.5, 1H), 5.55 (q, J= 7.0, 1H), 5.85 (m, 1H), 7.23-7.36 (m, 5H)
¹³C NMR (75 MHz, CDCl₃): 17.7, 22.5, 27.5, 39.0, 48.7, 50.5, 122.6, 126.9, 127.4, 128.6, 141.2, 158.9, 171.7

## Claims

1. A process for preparing a compound of formula (I), 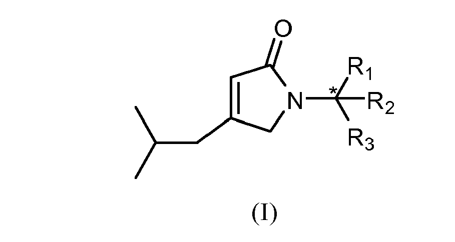 wherein R₁, R₂ and R₃ are each independently hydrogen, C₁-C₆ alkyl, C₃-C₆ cycloalkyl, aryl, heteroaryl, optionally substituted; which comprises the following steps:
a) transforming a compound of formula (VII) 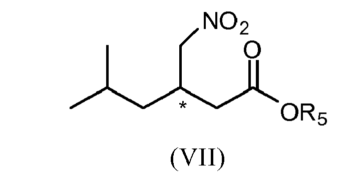 wherein R₅ is a C₁-C₄ alkyl group, or aryl, into an ester of formula (VI) 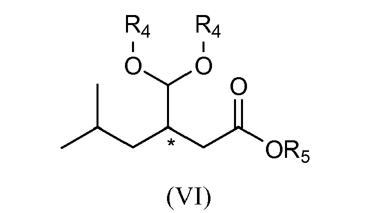 wherein R₄ is a C₁-C₆ alkyl group and R₅ is as defined above;
b) hydrolysing the ester of formula (VI) into a carboxylic acid of formula (III) 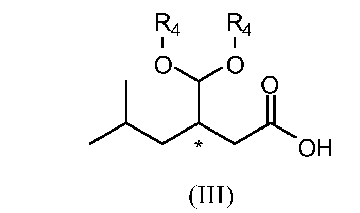 wherein R₄ is as defined above; 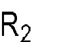
c) transforming the carboxylic acid of formula (III) into an amide of formula (II) 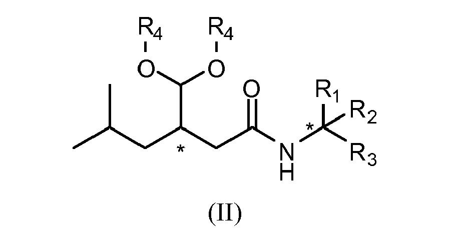 wherein R₁, R₂, R₃ and R₄ are as defined above;
d) cyclizing the amide of formula (II), into a compound of formula (I).

2. The process according to claim 1, wherein in step a) the compound of formula (VII) is transformed into an ester of formula (VI):
a) by addition of a base in an alcoholic solvent to form a nitronate salt;
b) adding the nitronate salt to a solution of a mineral acid in the alcoholic solvent;
c) neutralizing by addition of a base, removing the inorganic salts and optionally concentrating the solution of the ester of formula (VI).

3. The process according to claim 2, wherein the base is an alcoholate salt, such as sodium or potassium methoxide, sodium or potassium ethoxide, sodium or potassium t-butoxide.

4. The process according to claim 2, wherein said alcoholic solvent is methanol or ethanol.

5. The process according to claim 2, wherein the mineral acid is hydrochloric acid or sulfuric acid, preferably sulfuric acid.

6. The process according to claim 1, wherein in step b) the ester of formula (VI) is hydrolysed into a carboxylic acid of formula (III), by an alkaline hydrolysis reaction, followed by acidification and extraction in an inert solvent.

7. The process according to claim 1, wherein in step c) the carboxylic acid of formula (III) is transformed into an amide of formula (II):
a) by first reacting the carboxylic acid of formula (III) with a coupling agent, such as carbonyldiimidazole, dicyclohexyl carbodiimide, N-(3-dimethylaminopropyl)-N'-ethylcarbodiimide; or a carboxy-activating reagent acyl halide of formula (IIIa): 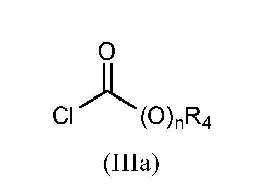 wherein R₄ is a C₁-C₆ alkyl group and n is 0 or 1; to obtain an activated derivative of formula (IV) 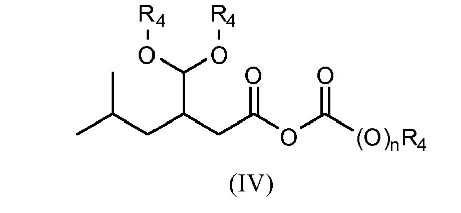 wherein R₄ and n are as defined above, in the presence of an organic base, in an inert solvent and, optionally, in the presence of an acylation catalyst, such as 4-(dimethylamino)pyridine;
b) and further reacting straight, without isolation, the compound of formula (IV) with a primary amine of formula (V) 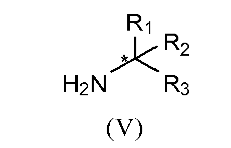
wherein R₁, R₂, R₃ are as defined in claim 1, optionally in the presence of an organic base, to give a compound of formula (II).

8. The process according to claim 7, step a) wherein the acyl halide is an acid chloride or a chloroformate.

9. The process according to claim 8, wherein the acid chloride is acetyl chloride, propionyl chloride or pivaloyl chloride, preferably pivaloyl chloride.

10. The process according to claim 8, wherein the chloroformate is ethyl chloroformate or isobutyl chloroformate, preferably ethyl chloroformate.

11. The process according to claim 7, wherein the organic base is a tertiary amine, such as triethylamine, tributylamine, *N*,*N*-diisopropylethylamine, *N*-methylmorpholine, preferably triethylamine.

12. The process according to claim 7, wherein the inert solvent is tetrahydrofuran.

13. The process according to claim 7, wherein the primary amine of formula (V) is benzylamine, (R)- or (S)-1-phenylethylamine, (R)- or (S)-1-(naphthalen-1-yl)ethylamine.

14. The process according to claim 1, step d) wherein the cyclization of a compound of formula (II) into a compound of formula (I) is carried out by reaction with an acid in a solvent.

15. The process according to claim 14, wherein the acid is a mineral acid, or an organic acid, carboxylic acid or a sulfonic acid, preferably a carboxylic acid or a sulfonic acid; more preferably a sulfonic acid; more and more preferably a camphorsulfonic acid.

16. The process according to claim 1, wherein R₁, R₂ and R₃ in formula (I) are each independently hydrogen, methyl, phenyl or 1-(naphthalen-1-yl).

17. The process according to claim 1, wherein the compounds of formula (I) are selected from:
(*R*)-4-isobutyl-1-(1-phenylethyl)-1H-pyrrol-2(5H)-one;
(*S*)-4-isobutyl-1-(1-phenylethyl)-1H-pyrrol-2(5H)-one;
(*R*)-4-isobutyl-1-(1-(naphthalen-1-yl)ethyl)-1H-pyrrol-2(5H)-one;
(*S*)-4-isobutyl-1-(1-(naphthalen-1-yl)ethyl)-1H-pyrrol-2(5H)-one.

18. A compound of formula (II), 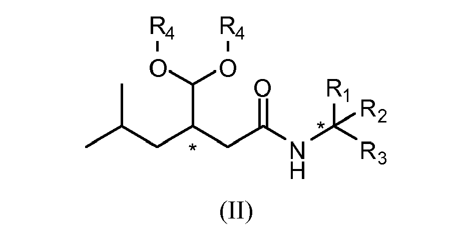 wherein:
R₁, R₂ and R₃ are each independently hydrogen, C₁-C₆ alkyl, C₃-C₆ cycloalkyl, aryl, heteroaryl, optionally substituted;
R₄ is a C₁-C₆ alkyl group; as well as its hydrates, solvates, racemic forms, diastereomers, and polymorphs, and mixtures thereof.

19. A compound according to claim 18, wherein R₁, R₂ and R₃ are each independently hydrogen, methyl, phenyl or 1-(naphthalen-1-yl).

20. A compound according to claim 18, wherein R₄ is methyl, ethyl or isopropyl.

21. A compound according to claim 18, selected from:
3-(dimethoxymethyl)-5-methyl-N-((*R*)-1-phenylethyl)hexanamide;
3-(diethoxymethyl)-5-methyl-N-((*R*)-1-phenylethyl)hexanamide;
3-(diisopropoxymethyl)-5-methyl-N-((*R*)-1-phenylethyl)hexanamide;
3-(dimethoxymethyl)-5-methyl-N-((*R*)-1-(naphthalen-1-yl)ethyl)hexanamide;
3-(diethoxymethyl)-5-methyl-N-((*R*)-1-(naphthalen-1-yl)ethyl)hexanamide;
3-(diisopropoxymethyl)-5-methyl-N-((*R*)-1-(naphthalen-1-yl)ethyl)hexanamide;
3-(dimethoxymethyl)-5-methyl-N-((*S*)-1-phenylethyl)hexanamide;
3-(diethoxymethyl)-5-methyl-N-((*S*)-1-phenylethyl)hexanamide;
3-(diisopropoxymethyl)-5-methyl-N-((*S*)-1-phenylethyl)hexanamide;
3-(dimethoxymethyl)-5-methyl-N-((*S*)-1-(naphthalen-1-yl)ethyl)hexanamide;
3-(diethoxymethyl)-5-methyl-N-((*S*)-1-(naphthalen-1-yl)ethyl)hexanamide;
3-(diisopropoxymethyl)-5-methyl-N-((*S*)-1-(naphthalen-1-yl)ethyl)hexanamide.

22. A compound of formula (III), 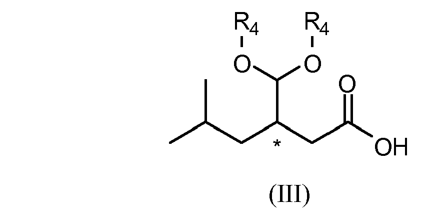 wherein R₄ is a C₁-C₆ alkyl group; as well as its hydrates, solvates, racemic forms, enantiomers, diastereomers, and polymorphs, and mixtures thereof, and the pharmaceutically acceptable salts thereof.

23. A compound according to claim 22, wherein R₄ is methyl, ethyl, isopropyl.

24. A compound according to claim 22, selected from:
3-(dimethoxymethyl)-5-methylhexanoic acid;
3-(diethoxymethyl)-5-methylhexanoic acid;
3-(diisopropoxymethyl)-5-methylhexanoic acid.

25. A compound of formula (VI), 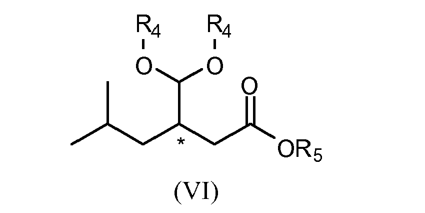 wherein R₄ is a C₁-C₆ alkyl group and R₅ is a C₁-C₄ alkyl group, or aryl; as well as its hydrates, solvates, racemic forms, enantiomers, diastereomers, and polymorphs, and mixtures thereof.

26. Use of compounds of formula (I), (II), (III) or (VI) as intermediates in the preparation of Pregabalin.
